# EUROPÄISCHE PATENTSCHRIFT

(11) **EP 1 152 780 B1**
(45) Veröffentlichungstag und Bekanntmachung des Hinweises auf die Patenterteilung: **23.11.2005**
(21) Anmeldenummer: 00906276.1
(22) Anmeldetag: 03.02.2000
(51) Int. Cl.: A61M 1/10, A61M 29/02, A61F 2/24, A61B 5/028

(54) **VORRICHTUNG ZUR INTRAVASALEN HERZKLAPPENOPERATION**
DEVICE FOR INTRAVASCULAR CARDIAC VALVE SURGERY
DISPOSITIF POUR OPERATION INTRAVASCULAIRE DE VALVULES CARDIAQUES

(30) Priorität: 06.02.1999 DE 19904975
(43) Veröffentlichungstag der Anmeldung: 14.11.2001
(73) Patentinhaber: Impella CardioSystems GmbH, 52074 Aachen (DE)
(72) Erfinder: SIESS, Thorsten, D-52146 Würselen (DE); FLAMENG, Willem, B-3000 Leuven (BE)
(74) Vertreter: Selting, Günther
(86) Internationale Anmeldenummer: PCT/EP2000/000863
(87) Internationale Veröffentlichungsnummer: WO 2000/045874

(56) Entgegenhaltungen:
- WO-A-91/17720
- WO-A-97/46270
- DE-A- 3 714 027
- DE-C- 19 613 565
- US-A- 4 819 751
- US-A- 5 746 709

## Beschreibung

Die Erfindung betrifft eine Vorrichtung zur intravasalen Verlegung einer Herzklappenprothese mit den Merkmalen des Anspruchs 1.

In WO 98/43688 ist eine intrakardiale Blutpumpe beschrieben, die einen Motorteil und einen Pumpenteil aufweist und durch das Blutgefäßsystem hindurch in das Herz eingeführt werden kann. Eine solche Blutpumpe kann beispielsweise durch die Aorta hindurch in der Aortenklappe positioniert werden, um Blut aus dem linken Ventrikel in die Aorta zu pumpen. Derartige Blutpumpen sind zur Unterstützung oder Ersetzung der Pumpwirkung des Herzens geeignet. Sie können im Herzen positioniert werden, ohne daß eine Öffnung des Herzens erforderlich ist.

Aus WO 97/37697 ist eine intravasale Blutpumpe bekannt, die ebenfalls einen Pumpenteil und einen Motorteil aufweist, welche mit einem Katheter verbunden sind. Die Blutpumpe kann durch das Blutgefäßsystem des Patienten geschoben werden. Es handelt es sich um eine Mikroaxialpumpe mit einem Durchmesser von maximal etwa 7 mm. Der Pumpenteil ist mit einer aufweitbaren Sperrvorrichtung umgeben, die den Strömungsweg außerhalb des Pumpengehäuses versperrt. Dadurch wird sichergestellt, daß das gesamte angesaugte Blut in Strömungsrichtung an dem Motorteil vorbei gefördert wird und Strömungskurzschlüsse um den Pumpenteil herum werden vermieden. Die Sperrvorrichtung kann aus einem ringförmigen Ballon bestehen, der an dem Pumpengehäuse oder an einem mit dem Pumpengehäuse verbundenen Ansaugschlauch befestigt ist. Diese Blutpumpe ist für den Einsatz in Blutgefäßen bestimmt, wobei die Sperrvorrichtung lediglich den Zweck hat, das Gefäßlumen um den Pumpenteil herum zu versperren, nicht aber die Gefäßwand zu deformieren. Demzufolge ist der Ballon der Sperrvorrichtung ein Niederdruckballon, der mit einem geringen Druck inflatiert wird, um gegen die Gefäßwand atraumatisch zu dichten.

Bekannt sind ferner Dilatationskatheter, die einen Ballon aufweisen, mit dem eine Gefäßstenose durch Aufdrücken beseitigt werden kann. Solche Dilatationskatheter können auch dazu benutzt werden, einen aus einem aufweitbaren Metallgestell bestehenden ringförmigen Stent, welcher eine Stützvorrichtung bildet, in das Blutgefäß einzubringen, um eine bleibende aufweitende Abstützung für die Stenose zu bilden. Ein solcher Dilatationskatheter ist in WO 97/46270 beschrieben. Dieser Dilatationskatheter ist als Perfusionskatheter ausgebildet, der ein Pumpenteil aufweist, um durch den inflatierten Ballon hindurch Blut zu fördern. Für eine Herzklappenoperation ist dieser Dilatationskatheter schon wegen seines geringen Durchmessers von maximal 2 mm nicht geeignet.

In US-A-5,746,709 ist eine intravasale Blutpumpe beschrieben, die von einem extrakorporalen Motor über eine biegsame Welle angetrieben ist. Der Pumpenteil weist einen Rotor auf, der in einem Pumpengehäuse rotiert. Das Pumpengehäuse wird von dem Innenrohr eines Ballonkatheters gebildet, so dass der Ballon unmittelbar auf dem Pumpengehäuse sitzt.

DE 196 13 565 C1 beschreibt eine intravasale Blutpumpe mit einem Antriebsteil, der einen Mikromotor zum Antreiben eines Pumpenteils aufweist. Auf dem Pumpengehäuse und einem damit verbundenen Ansaugschlauch sitzt ein ringförmiger Ballon, der im aufgeweiteten Zustand den Strömungsweg außerhalb des Pumpengehäuses versperrt und somit eine Sperrvorrichtung zur Verhinderung eines Bypassstroms bildet.

In US-A-4,819,751 ist ein Ballonkatheter zum Aufsprengen einer stenosierten Herzklappe beschrieben. Der Ballonkatheter weist einen hierfür geeigneten Ballon auf, der in der Herzklappe positioniert und dann aufgeweitet wird.

Die häufigsten Defekte an Herzklappen sind Klappeninsuffizienz und stenosierte Klappen. Bei Insuffizienz ist die Klappe nicht imstande vollständig zu schließen. Dies hat einen Rückstrom zur Folge. In der Regel müssen derartige Klappen durch künstliche Klappen ersetzt werden. Bei stenosierten Klappen sind die Klappensegel an den Rändern zusammengewachsen, wodurch sich die Klappe nur unvollständig öffnet und nicht den vollen Blutstrom durchläßt.

Es gibt zwei Formen der Operation an Herzklappen: Im Falle des Herzklappenersatzes wird die natürliche Herzklappe entnommen
und durch eine Bioprothese oder mechanische Herzklappe operativ ersetzt. Für diese Form der Operation ist es erforderlich, daß das Operationsfeld blutfrei gemacht wird, d.h. der natürliche Blutstrom umgelenkt wird. Im Falle einer Reparatur der natürlichen Herzklappe kann dagegen ein minimalinvasiver Eingriff mit Hilfe eines in die Klappe eingebrachten Ballons erfolgen. Für solche Vorgänge beispielsweise an der Aortenklappe ist großes Geschick des Herzchirurgen oder Kardiologen erforderlich. Dies liegt unter anderem daran, daß der Blutstrom durch die Aorta während des Sprengvorganges blockiert werden muß, so daß die Operation in kürzester Zeit durchgeführt werden muß, damit die Versorgung des Gefäßsystems wieder aufgenommen werden kann. Außerdem erhöht sich bei der Blockierung des Aortenstromes der Druck im Innern des Herzens extrem, während seine Versorgung mit Blut durch die Herzkranzgefäße zum Erliegen kommt.

Der Erfindung liegt die Aufgabe zugrunde, eine Vorrichtung zur intravasalen Herzklappenoperation zu schaffen, mit der minimalinvasive Klappenoperationen relativ einfach und ohne Zeitdruck vorgenommen werden können.

Die Lösung dieser Aufgabe erfolgt erfindungsgemäß mit den Merkmalen des Anspruchs 1.

Bei der erfindungsgemäßen Vorrichtung ist eine intravasale Mikroaxialpumpe vorgesehen, also eine Pumpe, die durch das Gefäßsystem des Patienten hindurchgeschoben werden kann und einen entsprechend geringen Außendurchmesser hat, der 8 mm nicht übersteigt. Der Pumpenteil der Mikroaxialpumpe trägt eine Dilatationsvorrichtung, die bei Positionierung in der Herzklappe zum Aufbrechen einer stenosierten Herzklappe imstande ist. Diese Dilatationsvorrichtung besteht vorzugsweise aus einem Hochdruckballon, dessen Durchmesser in aufgeweitetem Zustand mindestens 15 mm beträgt und der mit mindestens 1,0 bar inflatierbar ist.

Die erfindungsgemäße Vorrichtung dient zur intravasalen Verlegung einer Herzklappenprothese. Sie weist einen Stent auf, der an seiner Innenseite eine flexible Herzklappenprothese trägt. Der die Herzklappenprothese enthaltende Stent kann in die pathogene Herzklappe eingebracht und von der Dilatationsvorrichtung aufgeweitet werden, so daß er die Klappensegel der natürlichen Herzklappe auseinanderdrückt. Dabei wird die flexible Herzklappenprothese entfaltet. Diese Herzklappenprothese enthält eine ein- oder mehrflügelige Herzklappe, die dann automatisch in Funktion tritt und die natürliche Herzklappe ersetzt.

Als Herzklappenprothese kann eine von Kälbern oder Kühen entnommene Jugolarklappe verwendet werden. Hierbei handelt es sich um eine Bioprothese aus natürlichem Gewebe.

Der lichte Durchmesser des Pumpenteils bzw. der sich an den Pumpenteil anschließenden Kanüle beträgt mindestens 8 mm, um eine zu starke örtliche Blutströmung bei hohen physiologischen Volumenströmen von bis zu 7 l/min vermeiden. Außerdem sollte der Träger der Dilatationsvorrichtung einen Außendurchmesser von mindestens 8 mm haben, so daß eine hinreichend große ringförmige Stütze für die Dilatationsvorrichtung gebildet wird und das Ausmaß der Durchmesservergrößerung nicht zu groß wird.

Im folgenden werden unter Bezugnahme auf die Zeichnungen Ausführungsbeispiele der Erfindung näher erläutert.

Es zeigen:
- Fig. 1: eine in die Aortenklappe eingebrachte, nicht zur Erfindung gehörende, Vorrichtung zur Beseitigung einer Herzklappenstenose,
- Fig. 2: die Vorrichtung nach Fig. 1 bei aufgeweiteter Dilatationsvorrichtung,
- Fig. 3: einen Längsschnitt durch eine flexible Herzklappenprothese,
- Fig. 4: die Herzklappenprothese von Fig. 3 in Verbindung mit einem aufgeweiteten Stent,
- Fig. 5: einen Querschnitt des Pumpenteils mit einem umgebenden Ballon sowie einem Stent mit darin enthaltener ebenfalls gefalteter Herzklappenprothese,
- Fig. 6: einer Darstellung der erfindungsgemäßen Vorrichtung zum implantieren der flexiblen Herzklappenprothese, und
- Fig. 7: den Herzbereich mit implantierter Herzklappenprothese in Aortenposition.

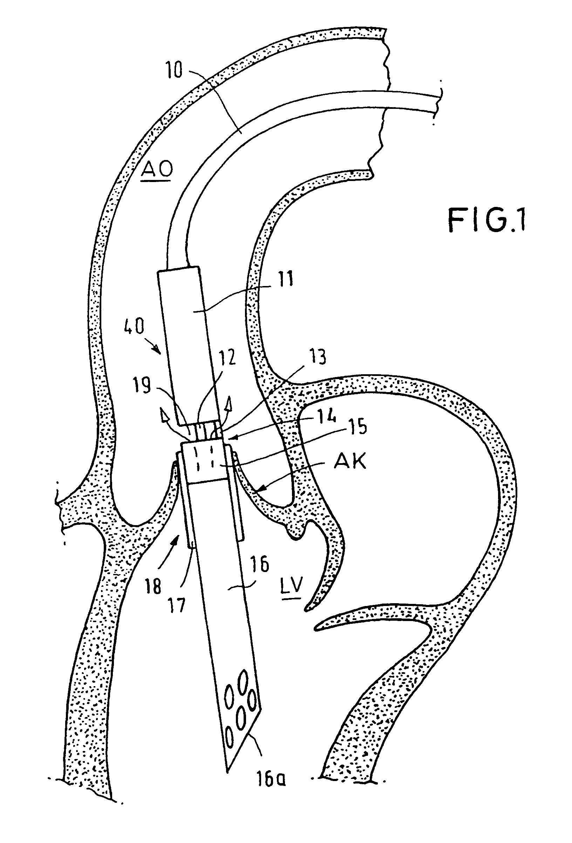

In Figur 1 ist ein Teil des Herzens dargestellt, nämlich der linke Ventrikel LV, aus dem das Blut durch die Aortenklappe AK hindurch in die Aorta AO strömt. Die Aortenklappe hat drei Klappensegel, die in die Aorta AO hinein vorstehen und sie bildet strömungstechnisch ein Rückschlagventil, das nur in die Aorta hinein durchlässig ist.

Zum Aufdrücken einer stenosierten Aortenklappe AK wird die in Fig. 1 dargestellte Dilatationsvorrichtung durch die Aorta hindurch in das Herz eingeführt. Diese Vorrichtung weist einen Katheter 10 auf, der mit einer Mikroaxialpumpe 40 verbunden ist. Diese weist einen Antriebsteil 11 auf, der einen Elektromotor enthält und eine Welle 12 antreibt, welche aus dem distalen Ende des zylindrischen Antriebsteils 11 herausragt. Von dem Antriebsteil 11 erstreckt sich ein Haltesteg 13 in axialer Richtung zu dem rohrförmigen Pumpenteil 14. Der Pumpenteil 14 besteht aus einem Pumpenring 15, welcher ein von der Antriebswelle 12 gedrehtes Flügelrad enthält, und einer den Pumpenring 15 axial fortsetzenden Kanüle 16. Die gesamte Pumpvorrichtung, nämlich der Antriebsteil 11 und der Pumpenteil 14, haben einen maximalen Durchmesser von 8 mm. Der Katheter 10 enthält die elektrischen Leitungen für die Versorgung und Steuerung der Mikroaxialpumpe 40 und ein Drucklumen, durch welches Druckflüssigkeit zugeführt werden kann.

Auf dem Pumpenteil 14 ist ein ringförmiger Ballon 17 angebracht, der in Fig. 1 im flachgelegten Zustand dargestellt ist. Der Ballon 17 kann durch das Drucklumen des Katheters 10 mit Flüssigkeit inflatiert werden. Es handelt sich um einen Hochdruckballon, dessen Durchmesser in aufgeweitetem Zustand mindestens 15 mm, vorzugsweise zwischen 15 und 40 mm, beträgt und der Drücken bis 8 bar standhält. Der Ballon 17 erstreckt sich über einen Teil der Länge des Pumpenringes 15 sowie auch über einen Teil der Länge der Kanüle 16. Er kann über seine gesamte Länge durch einen starren Ring abgestützt sein, der ein Zusammendrücken der Kanüle 16 verhindert.

Die Pumpvorrichtung wird in die Aorta eingeführt, indem zuerst ein (nicht dargestellter) Führungsdraht in der Aorta und den linken Ventrikel verlegt wird. Dann wird die Vorrichtung entlang des Führungdrahtes vorgeschoben und in der Aortenklappe AK so positioniert, daß der Ansaugbereich 16a sich im linken Ventrikel LV befindet, während der Ausstoßbereich 19 in der Aorta liegt. Der Pumpenteil 15 wird also von der Aortenklappe AK umschlossen. Die Pumpvorrichtung fördert rückwärts, d.h. sie saugt axial an und stößt im Ausstoßbereich 19 seitlich aus.

Nachdem die Pumpvorrichtung in der in Fig. 1 dargestellten Weise plaziert worden ist, wird der Antriebsteil 11 in Funktion gesetzt, so daß die Pumpe Blut von dem linken Ventrikel LV in die Aorta AO fördert. Dadurch wird das Herz volumen- und druckentlastet und das Herz beruhigt. Danach erfolgt das Inflatieren des Ballons 17, der die Dilatationsvorrichtung 18 bildet und mitten in der Aortenklappe AK aufgeweitet wird. Durch den mit hohem Druck inflatierten Ballon 17 werden die Klappensegel der Aortenklappe AK aufgedrückt und etwaige Anwachsungen an den Komissuren werden gesprengt. Auf diese Weise wird eine stenosierte Klappe so weit aufgedrückt, daß sie wieder den vollen Öffnungszustand einnehmen kann. Diese Form der Klappenoperation kann mit Hilfe der Pumpvorrichtung in einem beruhigten Umfeld stattfinden und ohne Hast durchgeführt werden, da das gesamte Herzminutenvolumen für die Zeit der Behandlung von der Pumpvorrichtung durch die Dilatationsvorrichtung gefördert wird. In vergleichbarer Weise kann mit der oben beschriebenen Dilationsvorrichtung die natürliche, stenosierte Mitralklappe gesprengt werden.

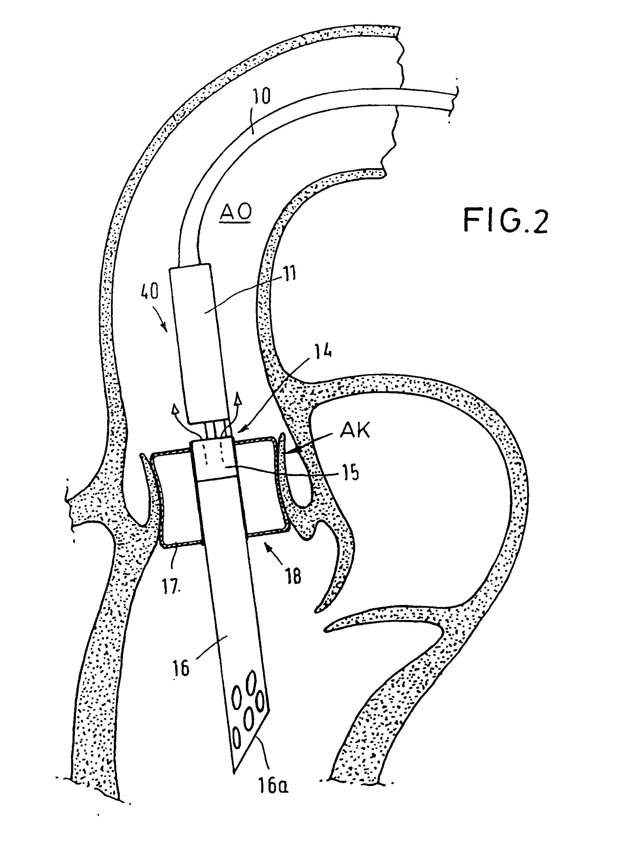

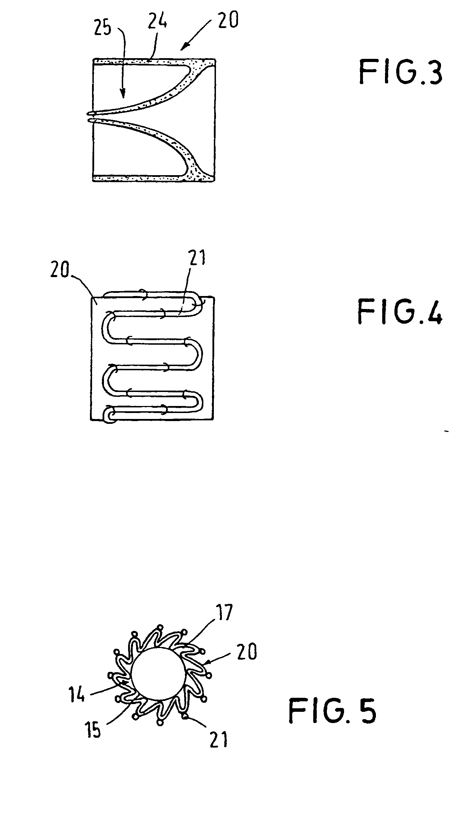

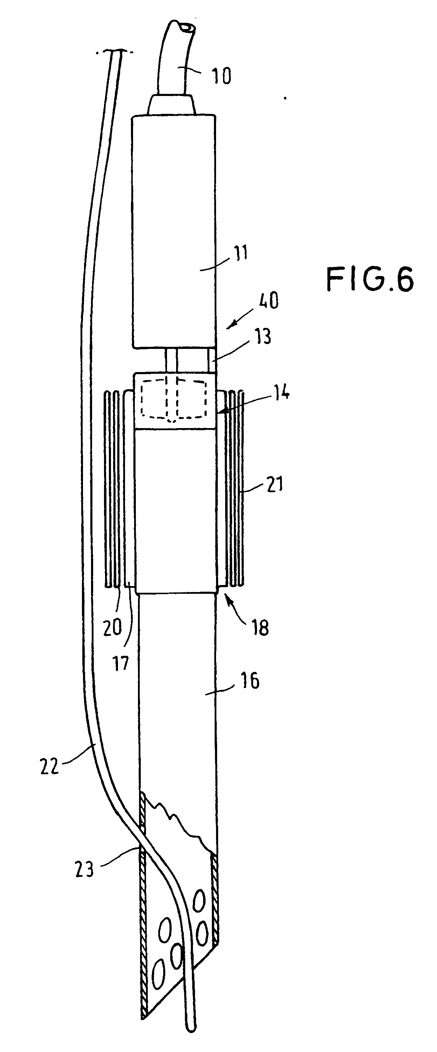

Die Figuren 3 bis 7 beziehen sich auf die erfindungsgemäße Vorrichtung, mit der eine insuffiziente Klappe durch eine Klappenprothese ersetzt wird. Hierzu wird generell die gleiche Vorrichtung angewandt, die anhand der Figuren 1 und 2 beschrieben wurde. Diese Vorrichtung ist in Figur 6 dargestellt. Auf der deflatierten und zusammengefalteten Dilatationsvorrichtung 18 sitzt eine flexible Herzklappenprothese 20 und darüber befindet sich ein spiralförmiger Stent 21 im zusammengedrückten Zustand.

In Figur 6 ist außerdem noch der Führungsdraht 22 dargestellt, welcher dazu dient, die Vorrichtung mit dem Katheter 10 vorzuschieben und lagerichtig zu plazieren. Der Führungsdraht 22 ragt am distalen Ende aus der Kanüle 16 heraus. Er führt durch ein seitliches Loch 23 der Kanüle und erstreckt sich dann außerhalb des Pumpenteils 14 und des Antriebsteils 11 entlang des Katheters 10.

Die flexible Herzklappenprothese 20 ist in Figur 3 dargestellt. Es handelt sich um eine Bioprothese, die einer Kuh oder einem Kalb entnommen wurde. Hierzu wurde ein Abschnitt 24 eines Blutgefäßes, der eine Gefäßklappe 25 enthält, herausgetrennt. Bei der Klappe 25 kann es sich um eine einflügelige oder dreiflügelige Klappe handeln. Diese Klappenprothese 20 wird gemäß Figur 4 in einen Stent 21 eingesetzt. Der Stent 21 ist ein rohrförmiges Element aus Metallstäben, die hier mäanderförmig gebogen sind und es ermöglichen, daß der Stent axial zusammengedrückt oder radial aufgeweitet wird. Es können auch andere Stentstrukturen verwandt werden, wie beispielsweise eine Zellenstruktur aus Streckmaterial. Wichtig ist, daß der Stent eine komprimierte Rohrform und eine expandierte Rohrform stabil einnehmen kann. An den Stäben des Stents 21 ist die schlauchförmige Wand 24 der Gefäßprothese 20 angenäht. Die Gefäßprothese 20 ist somit auf der Innenseite des Stents 21 befestigt, so daß nach Implantation der Gefäßprothese der Stent zwischen der natürlichen Herzklappe und der Bioprothese zu Liegen kommt und keinen Kontakt zum Blut hat. Somit erwächst aus dem Stent nicht die Notwendigkeit, Antikoagulatien gegen die Bildung von Thromben geben zu müssen.

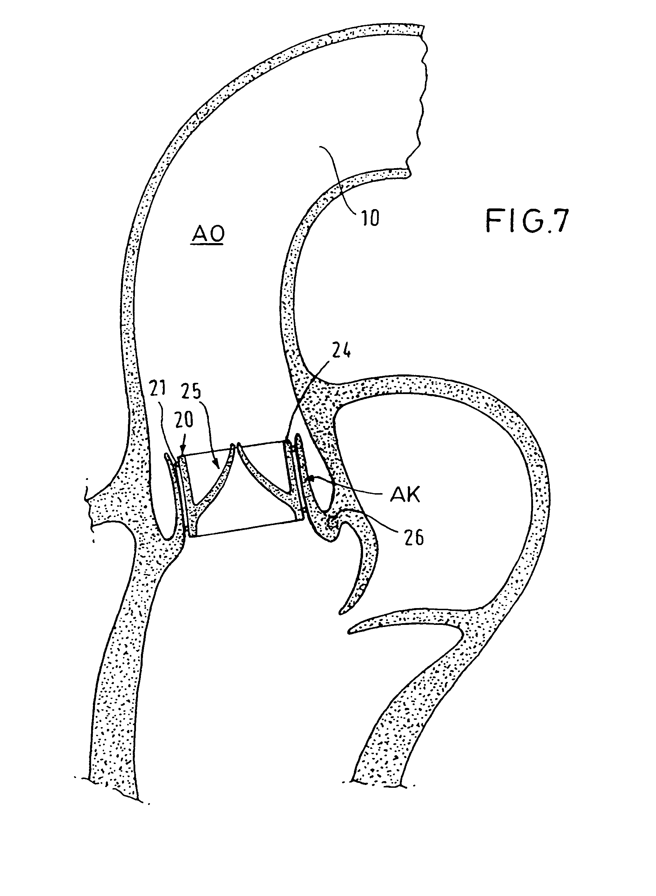

In Figur 5 ist der Pumpenteil 14 mit dem Pumpenring 15 dargestellt. Um den Pumpenring 15 sind der Ballon der Dilatationsvorrichtung 18 und die Klappenprothese 20 in Form von zahlreichen Schleifen gefaltet, wobei die Klappenprothese an den Stäben des Stents 21 befestigt ist. In diesem Zustand bilden der Ballon, die Klappenprothese 20 und der Stent 21 ein flaches Paket, das den Pumpenteil 14 umgibt. Dieses Paket wird in der natürlichen Herzklappe AK positioniert. Danach wird die Pumpe in Betrieb gesetzt und dann wird bei laufender Pumpe die Dilatationsvorrichtung 17 aufgepumpt. Dabei wird der Stent 21 dilatiert, wobei er gemäß Fig. 7 die Herzklappenprothese 24 aufweitet und die Segel der natürlichen Aortenklappe AK nach außen in die geöffnete Position (systolische Klappenposition) drückt.

Dadurch wird die Aortenklappe AK außer Funktion gesetzt. Der Stent 21 verbleibt in der Herzklappenöffnung. In ihm befindet sich die Herzklappenprothese 24, die auf ihren Originalzustand aufgeweitet wurde und in der sich die Klappensegel 25 befinden. Diese Herzklappe übernimmt nun die Funktion der natürlichen Aortenklappe AK.

Zur Vermeidung von Verschiebungen der Herzklappenprothese kann der Stent 21 oder die Herzklappenprothese 20 in dem Anulus 26 fixiert werden, der die natürliche Herzklappe umgibt. Es handelt sich um einen festen Knorpelring, der als Halterung für eine Herzklappenprothese geeignet ist.

## Patentansprüche

1. Vorrichtung zur intravasalen Herzklappenoperation, mit einer an einem Katheter (10) befestigten Mikroaxialpumpe (40), die einen rohrförmigen Pumpenteil (14) aufweist, und einer Dilatationsvorrichtung (18) zum Aufweiten eines Stents (21),
**gekennzeichnet durch** einen
von der Dilatationsvorrichtung (17) aufweitbaren Stent (21), der eine gefaltete flexible Herzklappenprothese (20) trägt.

2. Vorrichtung nach Anspruch 1, wobei die Herzklappenprothese (20) an den Stent (21) angenäht ist.

3. Vorrichtung nach Anspruch 1 oder 2, wobei die Herzklappenprothese (20) eine schlauchförmige Wand (24) aufweist, die zusammen mit einer Ballonwand der Dilatationsvorrichtung (17) gefaltet ist.

## Claims

1. A device for intravascular cardiac valve surgery, comprising a microaxial pump (40) fastened to a catheter (10) and having a tubular pump portion (14), and a dilating device (18) for widening a stent (21),
**characterized by** a stent (21) adapted to be widened by the dilating device (17) and carrying a folded flexible cardiac valve prosthesis (20).

2. The device according to claim 1 wherein the cardiac valve prosthesis (20) is sewn to the stent (21).

3. The device according to claim 1 or 2 wherein the cardiac valve prosthesis (20) comprises a tubular wall (24) which is folded together with a balloon wall (17) of the dilating device.

## Revendications

1. Dispositif permettant l'opération intravasculaire de valvules cardiaques, doté d'une micropompe (40) fixée à un cathéter (10) présentant une partie de pompage tubulaire (14), et d'un dispositif de dilatation (18) pour élargir un stent (21),
**caractérisé par** un stent (21) pouvant être élargi par un dispositif de dilatation (17) qui porte une prothèse valvulaire cardiaque flexible (20) pliée.

2. Dispositif selon la revendication 1, dans lequel la prothèse valvulaire cardiaque (20) est cousue sur le stent (21).

3. Dispositif selon la revendication 1 ou 2, dans lequel la prothèse valvulaire cardiaque (20) présente une paroi tubulaire (24) qui est pliée conjointement avec une paroi de ballonnet du dispositif de dilatation (17).
